(19) Europäisches Patentamt
European Patent Office
Office européen des brevets

(11) **EP 1 941 052 B1**

(12) **EUROPEAN PATENT SPECIFICATION**

(45) Date of publication and mention
of the grant of the patent:
**18.12.2013 Bulletin 2013/51**

(21) Application number: **06801614.6**

(22) Date of filing: **15.08.2006**

(51) Int Cl.:
***G01N 31/00*** *(2006.01)*

(86) International application number:
**PCT/US2006/031964**

(87) International publication number:
**WO 2007/035217 (29.03.2007 Gazette 2007/13)**

(54) **METHOD FOR REGENERATING HYDROPHILIC AND OSTEOPHILIC SURFACES OF AN IMPLANT**

VERFAHREN ZUR REGENERIERUNG DER HYDROPHILEN UND OSTEOPHILEN OBERFLÄCHE EINES IMPLANTATS

PROCÉDÉ PERMETTANT DE REGÉNÉRER LA SURFACE HYDROPHILE ET OSTÉOPHILE D'UN IMPLANT

(84) Designated Contracting States:
**AT BE BG CH CY CZ DE DK EE ES FI FR GB GR HU IE IS IT LI LT LU LV MC NL PL PT RO SE SI SK TR**

(30) Priority: **20.09.2005 US 719262 P**

(43) Date of publication of application:
**09.07.2008 Bulletin 2008/28**

(73) Proprietor: **The Regents of the University of California**
**Oakland, CA 94607-5200 (US)**

(72) Inventor: **OGAWA, Takahiro**
**Torrance, CA 90503 (US)**

(74) Representative: **Gervasi, Gemma**
**Notarbartolo & Gervasi GmbH**
**Bavariaring 21**
**80336 München (DE)**

(56) References cited:
**WO-A1-00/44305     WO-A1-03/030957**

• **BAGNO ET AL.: 'Surface Treatment and Roughness Properties ofTi-based Biomaterials.' JOURNAL OF MATERIALS SCIENCE: MATERIALS IN MEDICINE. vol. 15, 2004, pages 935 - 949, XP019212062**

**Description**

BACKGROUND OF THE INVENTION

Field of the Invention

This invention generally relates to method for regenerating hydrophilic and osteophilic surface on an implant implant that has a hydrophobic or non-osteophilic surface and to a kit capable of regenerating a hydrophilic surface on an implant.

Description of the Background

Restoration of skeletal defects or wounds such as femoral neck fracture and spine fusion are common procedures. For example, over 500,000 and 250,000 procedures are performed annually in the U.S. for hip prosthesis implantation and spine fusion surgery, respectively. Meanwhile, about 74 million people in the U.S., which amounts to about 30% of adult population in the U.S., have at least one qradrant of posterior missing tooth that needs to be restored.

Some metallic materials such as titanium are proven biocompatible materials. For example, use of titanium implants has become a standard treatment to replace missing teeth and to fix diseased, fractured or transplanted bone. Restorative treatment of missing teeth using dental implants such as titanium implants have considerable oral health impact, by which masticatory function (Carlsson GE, Lindquist LW, Int. J. Prosthodont 7 (5):448-53 (1994); Geertman ME, et al., Community Dent Oral Epidemiol 24(1):79-84 (1996); Pera P, et al., J Oral Rehabil 25(6):462-7 (1998); van Kampen FM, et al., J Dent Res 83(9):708-1 1 (2004)), Speech (Heydecke G, et al., J Dent Res 83(3):236-40 (2004)) and daily performance and quality of life (Melas F, et al., Int J Oral Maxillofac Implants 16(5):700-12 (2001)) are improved, when compared to the conventional removable denture treatment. In treatments of facial defect resulting from cancer or injury, the use of endosseous implants is crucial to retain the prosthesis (Roumanas ED, et al., Lit J Prosthodont 15(4): 325-32 (2002)). However, the application of implant therapy in these fields is still limited because of various risk factors including anatomy and quality of host bone (van Steenberghe D, et al., Clin Oral Implants Res 13(6):617-22 (2002)), systemic conditions including diabetes (Nevins ML, Int J Oral Maxillofac Implants 13 (5):620-9 (1998); Takeshita F, et al., J Periodontal 69(3): 314-20 (1998) and osteoporosis (Ozawa S, et al., Bone 30(1): 137-43 (2002)), and ageing (Takeshita F, et al., J Biomed Mater Res 34(1):1-8 (1997)). More importantly, long healing time (about 4-10 months) required for titanium implants to integrate with surrounding bone restricts the application of this beneficial treatment. For example, in the U.S., dental implant therapy has penetrated into only 2% of the potential patients.

In the orthopedic field, the restoration of femoral neck fracture or spine fusion, for example, is a common problem. For example, of over 250,000 procedures performed annually in the U.S. for spine fusion surgery, about 30% or more of patients fail to achieve a solid bony union. The nature and location of bone fracture at these areas do not allow for bone immobilization (e.g., cast splinting) for better healing.

Despite the growing needs of titanium implants, a decent percentage of unsuccessful implants, for instance, ranging 5%-40% in orthopedic implants, and limited application and protracted healing time of implants, particularly in dental implants, are the immediate challenges. Furthermore, implant placements often times have the impaired bone regenerative potential, such as osteoporotic and aged metabolic properties, and thus can lead to difficulty in achieving biological requirements of bone-titanium integration (see, e.g., Ozawa, S. et al. Bone 30, 137-43 (2002); Zhang, H., et al.; J Orthop Res 22, 30-8 (2004); Takeshita, F., et al., J Biomed Mater Res 34, 1-8 (1997); and Yamazaki, M. et al. Oral Surg Oral Med Oral Pathol Oral Radiol Endod 87, 411-8 (1999)).

Freshly prepared titanium surfaces are known to be hydrophilic, while the ones exposed to atmosphere and other liquid for long time become hydrophobic. A hypothetical mechanism is that progressive contamination of carbon and hydrocarbon to the titanium surfaces alters the wettability behavior. Those contaminants can be absorbed onto the titanium surface from the atmosphere and various cleaning solution such as methanol and acetone. However, the osteoconductive potentials, which are crucial for successful implants, in association with the changes in hydrophilicity behavior of implants are unknown and may be reduced with the reduction of hydrophilicity (please see WO 0044305 and WO 03030957)..

Therefore, there is a need for testing the aging of an implant. There is also a need for a method for regenerating hydrophilic as well as osteophilic metallic implant surface.

The embodiments described below address the above identified issues and needs.

SUMMARY OF THE INVENTION

The present invention provides a method for regenerating a hydrophilic or osteophilic surface according to claim 1.

The present invention further provides Kits capable of regenerating a hydrophilic surface according to claim 5.

BRIEF DESCRIPTION OF DRAWINGS

Figures IA- 1C (not according to the present invention) show study on the hydrophilicity of the freshly prepared titanium surface and the aged titanium surfaces. In Figure IA, the freshly prepared sandblasted surface, 2 week-old sandblasted surface and machined surface were compared. Figures IB and 1C show the test results on their respective time-related attenuating change in hydrophilicity of surfaces prepared by acid-etching and machining.

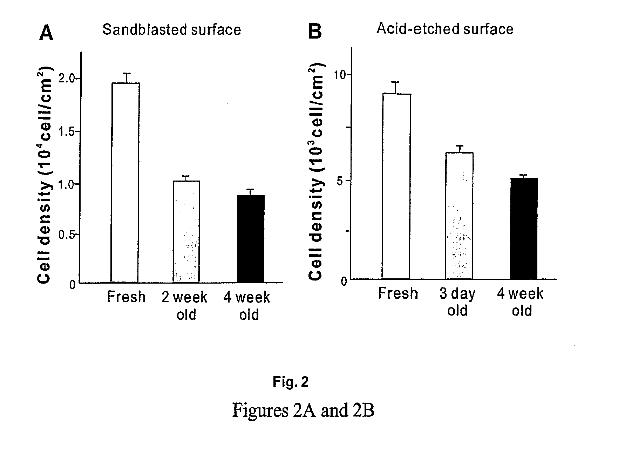

Figures 2A-B (not according to the present invention) show the cell proliferation of osteoblasts on the freshly prepared titanium surfaces and the aged surfaces.

DETAILED DESCRIPTION

Relative to implants with a hydrophobicity surface (aged implant), implants with a hydrophilic surface have an enhanced (faster and stronger) tissue integration (osteoconduction) capability. The present invention provides a method for regenerating a hydrophilic and osteophilic surface on an aged implant that has a hydrophobic or osteophobic surface according to claim 1. The method includes substantially breaking the hydrophobic and osteophobic surface, and removing the hydrophobic and osteophobic surface from the implant or altering the physicochemical properties of the surface.

Kits for regenerating a hydrophilic and osteophobic surface on an implant according to claim 5 are also provided herein. The kit can be a stand-alone kit or can be included in a product package that includes the implant.

As used herein, the term "hydrophilicity" refers to an attribute of a material that defines the degree of water affinity of surface of the implant. Hydrophobicity and hydrophilicity are relative terms. Generally, hydrophobicity and hydrophilicity of a surface can be gauged using the Hildebrand solubility parameter [delta]. The term "Hildebrand solubility parameter" refers to a parameter indicating the cohesive energy density of a substance. The [delta] parameter is determined as follows:

$$\delta = (\Delta E/V)^{1/2}$$

where $\delta$ is the solubility parameter, $(cal/cm^3)^{1/2}$;

$\Delta E$ is the energy of vaporization, cal/mole; and

V is the molar volume, $cm^3$/mole.

The term "tissue integration capability" refers to the ability of a medical implant to be integrated into the tissue of a biological body through its osteoconductive process. The tissue integration capability of an implant can be generally measured by several factors, one of which is wettability of the implant surface, which reflects the hydrophilicity/oleophilicty (hydrophobicity), or hemophilicity of an implant surface. Hydrophilicity and oleophilicity are relative terms and can be measured by, e.g., water contact angle (Oshida Y, et al., J Mater Science 3:306-312 (1992)), and area of water spread (Gifukosen on line text, http://www.gifu-nct.ac.jp/elec/tokoro/fft/contact-angle.html). For purposes of the present invention, the hydrophilicity/oleophilicity can be measured by contact angle or area of water spread on an implant surface described herein relative to the ones of the control implant surfaces. Relative to the implant surfaces not treated with the process described herein, a medical implant treated with the process described herein has a substantially lower contact angle or a substantially higher area of water spread.

Methods for regenerating hydrophilic and osteophilic surface

According to the present invention a method for regenerating hydrophilic and osteophilic surface of an implant according to claim 1 is provided.. In some embodiments, the present invention provides a kit for regenerating a hydrophilic and osteophilic surface of an implant according to claim 1. The kit includes a device capable of removing the aged surface layer from or breaking the aged surface layer on an implant so as to regenerate a hydrophilic and osteophilic surface on the implant characterized in that the device is sandpaper or a file.

In some embodiments, the kit can be provided along with the implant or in a separate package.

Example (not according to the present invention). Degrading osteoinductive potential of titanium over time and test for titanium aging and quality control of titanium implants Materials and methods

Titanium sample. Disks (20mm in diameter and 1.5mm in thickness) made of commercially pure titanium (Grade 2) were used. The surface of the disks were freshly prepared by machine turning, sandblasting of 50 $\mu$m aluminum oxide particles at a distance of 1 cm with a pressure of 3 kg/m, and acid-etching with $H_2SO_4$.

Hydrophilicity testing. Ten $\mu$l of distilled water was gently placed on the titanium surface without physical contact and digitally photographed immediately. The spread area was measured as the area of the water drop in the top view using a digital analyzer (Image Pro Plus, Media Cybernetics, Silver Spring, MD). The contact angle $\theta$ were obtained by the equation: $\theta = 2\tan^{-1}(2h/d)$, where h and d are the height and diameter of the drop in the side view (Oshida, Y. et al., J Mater Science 3, 306-312 (1992)).

Osteoblastic Cell Culture. Bone marrow cells isolated from the femur of 8-week-old male Sprague-Dawley rats were placed into alpha-modified Eagle's medium supplemented with 15% fetal bovine serum, 50mg/ml ascorbic acid, $10^{-8}$M dexamethasone, 10mM Na-$\beta$-glycerophosphate and Antibiotic-antimycotic solution containing 10000 units/ml Penicillin G sodium, 10000 mg/ml Streptomycin sulfate and 25 mg/ml Amphotericin B. Cells were incubated in a humidified atmosphere of 95% air, 5% $CO_2$ at 37°C. At 80% confluency, the cells were detached using 0.25% Trypsin- 1mM EDTA-4Na and seeded onto either the machined titanium or acid-etched titanium disks at a density of 5x $10^4$ cells/cm$^2$. The culture medium was renewed every three days.

Proliferation assay. To examine the cell proliferation, the osteoblastic cells were incubated on the titanium discs placed on the polystyrene culture dish. The freshly prepared disks and one aged disks of various time periods were used. The cells were gently rinsed twice with PBS and treated with 0.1% collagenase in 300 $\mu$l of 0.25% trypsin-1mM EDTA- 4Na for 15 min at 37°C. A hematocytometer was used to count the number of detached cells.

Statistical Analysis. ANOVA was used to examine differences in wettability and cell proliferation variables between the freshly prepared titanium surfaces and aged surfaces; <0.05 was considered statistically significant.

Results

Superhydrophilicity of fresh titanium surface and its reduction with time. Sandblasting on the machined surface changed the wettability behavior from hydrophobic to hydrophilic (Figure IA). The spread area of 10 $\mu$l water drop increased 13 times after sandblasting of the machined surface. The contact angle of water before sandblasting, which was 69.9°, plummeted to 2.0° after sandblasting, indicating the generation of super- hydrophilic surfaces. The created superhydrophilic property was reduced to 50% level after two weeks in terms of the spread area. The contact angle was increased accordingly with time.

The freshly prepared acid-etched surface and machined surface also showed the superhydrophilic property, which was faded out with time (Figures IB and C). After 4 weeks, the hydrophilic properties at the fresh stage were changed to hydrophobic nature for the both surface types.

Reducing cell proliferation of osteoblasts on ageing titanium. The freshly prepared titanium surfaces showed highest cell proliferation for the different surface types. The cell proliferation was significantly reduced with time for the different surface types (p<0.0001) (Figures 2A and 2B). The proliferation was reduced to approximately 50% on the 4 week-old surface compared with the freshly prepared surfaces.

**Claims**

1. A method for regenerating a hydrophilic or osteophilic surface on an implant that has a hydrophobic or non-osteophilic surface, comprising using sandpaper or a file to break or substantially remove the hydrophobic or non-osteophilic surface so as to regenerate the hydrophilic or osteophilic surface.

2. The method of claim 1, further comprising irradiation with ultraviolet light.

3. The method of claim 1 wherein the implant is a metallic implant, preferably a titanium implant, or an implant comprising titanium, gold, platinum, tantalum, niobium, nickel, iron, chromium, cobalt, zirconium, magnesium, aluminum, palladium, or an alloy formed thereof.

4. The method of claim 1, wherein the implant is a non-metallic implant.

5. A kit capable of regenerating a hydrophilic surface on an implant, comprising a device or an agent capable of substantially breaking or removing a hydrophobic surface on an implant, **characterized in that** the device is sandpaper or a file, and wherein the kit is in a package comprising the implant.

6. The kit of claim 5 wherein the device is a file.

7. The kit of claim 5, comprising an etching agent, wherein the etching agent is an acid or a base.

8. The kit of claim 5, wherein the implant is a metallic implant or a non-metallic implant, preferably wherein the metallic implant comprises titanium, gold, platinum, tantalum, niobium, nickel, iron, chromium, cobalt, zirconium, magnesium, aluminum, palladium, or an alloy formed thereof.

**Patentansprüche**

1. Verfahren zur Regenerierung einer hydrophilen oder osteophilen Oberfläche auf einem Implantat, welches eine hydrophobe oder nicht-osteophile Oberfläche aufweist, wobei dieses Verfahren die Verwendung von Sandpapier oder einer Feile umfasst, um die hydrophobe oder nicht-osteophile Oberfläche aufzubrechen oder im Wesentlichen zu entfernen, so dass die hydrophile oder osteophile Oberfläche regeneriert wird.

2. Verfahren nach Anspruch 1, welches ferner das Bestrahlen mit ultraviolettem Licht umfasst.

3. Verfahren nach Anspruch 1, bei welchem das Implantat ein metallisches Implantat, vorzugsweise ein Titan-Implantat oder ein solches Implantat ist, welches Titan, Gold, Platin, Tantal, Niob, Nickel, Eisen, Chrom, Kobalt, Zirkonium, Magnesium, Aluminium, Palladium oder eine aus diesen bestehende Legierung enthält.

4. Verfahren nach Anspruch 1, bei welchem das Implantat ein nichtmetallisches Implantat ist.

**5.** Ausrüstungssatz, welcher zur Regenerierung einer hydrophilen Oberfläche auf einem Implantat geeignet ist und welcher eine Vorrichtung oder ein Mittel umfasst, welches zum wirksamen Aufbrechen oder Entfernen einer hydrophoben Oberfläche auf einem Implantat geeignet ist, **dadurch gekennzeichnet, dass** es sich bei der Vorrichtung um Sandpapier oder eine Feile handelt, und wobei dieser Ausrüstungssatz sich in einer Packung befindet, welche das Implantat umfasst.

**6.** Ausrüstungssatz nach Anspruch 5, wobei die Vorrichtung eine Feile ist.

**7.** Ausrüstungssatz nach Anspruch 5, welcher ein ätzendes Mittel umfasst, wobei dieses ätzende Mittel eine Säure oder eine Lauge ist.

**8.** Ausrüstungssatz nach Anspruch 5, bei welchem das Implantat ein metallisches Implantat oder ein nichtmetallisches Implantat ist, wobei vorzugsweise das metallische Implantat Titan, Gold, Platin, Tantal, Niob, Nickel, Eisen, Chrom, Kobalt, Zirkonium, Magnesium, Aluminium, Palladium oder eine aus diesen bestehende Legierung umfasst.

**Revendications**

**1.** Procédé de régénération d'une surface hydrophile ou ostéophile d'un implant qui a une surface hydrophobe ou non ostéophile, comprenant l'utilisation de papier de verre ou d'une lime pour casser ou sensiblement éliminer la surface hydrophobe ou non ostéophile de façon à régénérer la surface hydrophile ou ostéophile.

**2.** Procédé selon la revendication 1, comprenant en outre le rayonnement avec une lumière ultraviolette.

**3.** Procédé selon la revendication 1, dans lequel l'implant est un implant métallique, de préférence un implant en titane ou un implant comprenant du titane, de l'or, du platine, du tantale, du niobium, du nickel, du fer, du chrome, du cobalt, du zirconium, du magnésium, de l'aluminium, du palladium ou un alliage formé à partir de ceux-ci.

**4.** Procédé selon la revendication 1, dans lequel l'implant est un implant non métallique.

**5.** Kit de régénération d'une surface hydrophile d'un implant, comprenant un dispositif ou un agent pouvant sensiblement casser ou éliminer une surface hydrophobe d'un implant, **caractérisé en ce que** le dispositif est du papier de verre ou une lime et dans lequel le kit est dans un emballage comprenant l'implant.

**6.** Kit selon la revendication 5, dans lequel le dispositif est une lime.

**7.** Kit selon la revendication 5, comprenant un agent décapant, dans lequel l'agent décapant est un acide ou une base.

**8.** Kit selon la revendication 5, dans lequel l'implant est un implant métallique ou un implant non métallique, de préférence dans lequel l'implant métallique comprend du titane, de l'or, du platine, du tantale, du niobium, du nickel, du fer, du chrome, du cobalt, du zirconium, du magnésium, de l'aluminium, du palladium ou un alliage formé à partir de ceux-ci.

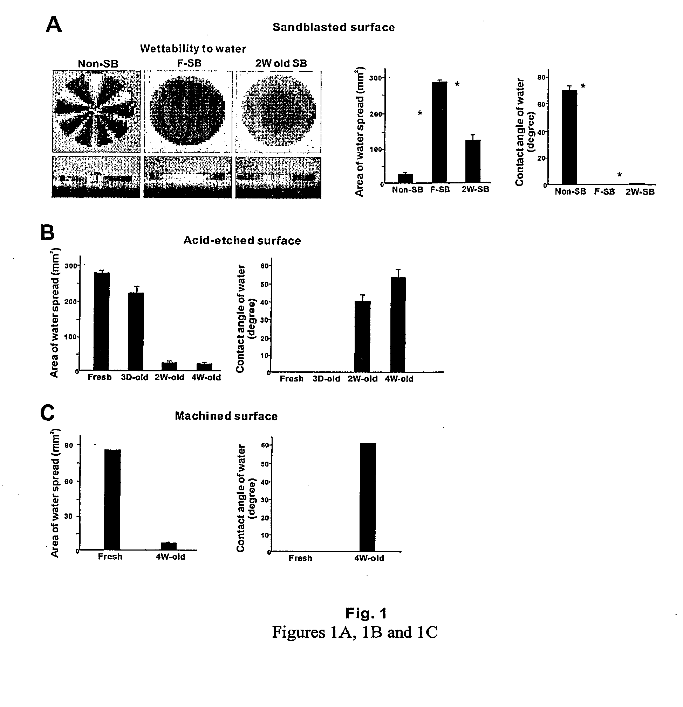

**Fig. 1**

Figures 1A, 1B and 1C

**A** Sandblasted surface   **B** Acid-etched surface

**Fig. 2**

Figures 2A and 2B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 0044305 A
- WO 03030957 A

### Non-patent literature cited in the description

- **CARLSSON GE ; LINDQUIST LW.** *Int. J. Prosthodont,* 1994, vol. 7 (5), 448-53
- **GEERTMAN ME et al.** *Community Dent Oral Epidemiol,* 1996, vol. 24 (1), 79-84
- **PERA P et al.** *J Oral Rehabil,* 1998, vol. 25 (6), 462-7
- **VAN KAMPEN FM et al.** *J Dent Res,* 2004, vol. 83 (9), 708-1 1
- **HEYDECKE G et al.** *J Dent Res,* 2004, vol. 83 (3), 236-40
- **MELAS F et al.** *Int J Oral Maxillofac Implants,* 2001, vol. 16 (5), 700-12
- **ROUMANAS ED et al.** *Lit J Prosthodont,* 2002, vol. 15 (4), 325-32
- **VAN STEENBERGHE D et al.** *Clin Oral Implants Res,* 2002, vol. 13 (6), 617-22
- **NEVINS ML.** *Int J Oral Maxillofac Implants,* 1998, vol. 13 (5), 620-9
- **TAKESHITA F et al.** *J Periodontal,* 1998, vol. 69 (3), 314-20
- **OZAWA S et al.** *Bone,* 2002, vol. 30 (1), 137-43
- **TAKESHITA F et al.** *J Biomed Mater Res,* 1997, vol. 34 (1), 1-8
- **OZAWA, S. et al.** *Bone,* 2002, vol. 30, 137-43
- **ZHANG, H. et al.** *J Orthop Res,* 2004, vol. 22, 30-8
- **TAKESHITA, F. et al.** *J Biomed Mater Res,* 1997, vol. 34, 1-8
- **YAMAZAKI, M. et al.** *Oral Surg Oral Med Oral Pathol Oral Radiol Endod,* 1999, vol. 87, 411-8
- **OSHIDA Y et al.** *J Mater Science,* 1992, vol. 3, 306-312
- **OSHIDA, Y. et al.** *J Mater Science,* 1992, vol. 3, 306-312